Europäisches Patentamt

European Patent Office

Office européen des brevets

(11) Veröffentlichungsnummer: **0 275 535**
**A1**

(19)

(12) **EUROPÄISCHE PATENTANMELDUNG**

(21) Anmeldenummer: 87119076.5

(51) Int. Cl.⁴: **A61F 2/24**

(22) Anmeldetag: 22.12.87

(30) Priorität: 23.12.86 US 946385

(43) Veröffentlichungstag der Anmeldung:
27.07.88 Patentblatt 88/30

(84) Benannte Vertragsstaaten:
AT BE CH DE ES FR GB GR IT LI LU NL SE

(71) Anmelder: **Baykut, Doguhan, Dr. med.**
**Senator-Balcke-Strasse 56**
**D-2800 Bremen 1(DE)**

(72) Erfinder: **Baykut, Doguhan, Dr. med.**
**Senator-Balcke-Strasse 56**
**D-2800 Bremen 1(DE)**

(74) Vertreter: **Eisenführ & Speiser**
**Martinistrasse 24**
**D-2800 Bremen 1(DE)**

(54) **Herzklappenprothese.**

(57) Die Erfindung betrifft eine Herzklappenprothese in Form eines Zweiwegeventils mit einem im wesentlichen rohrartigen Ventilkörper (10, 50), der mindestens eine erste Durchtrittsöffnung definiert, und einem mit diesem verbundenen Ventilelement, das unter dem Richtungswechsel des Blutdrucks zwischen einer die Durchtrittsöffnung verschließenden Sperrstellung und einer sie freigebenden Öffnungsstellung bewegbar ist und das Blut in der Sperrstellung zu einer zweiten Durchtrittsöffnung (40) gelangen läßt. Das Ventilelement wird von einer flexiblen Zunge (20, 60) gebildet, die nur an ihrem einen Endbereich (22) mit einem Umfangs-Teilbereich des Ventilkörpers (10, 50) verbunden ist.

Figur 5

Figur 6

EP 0 275 535 A1

## Herzklappenprothese

Die Erfindung betrifft eine Herzklappenprothese in Form eines Zweiwegeventils, mit einem im wesentlichen rohrartigen Ventilkörper, der mindestens eine erste Durchtrittsöffnung definiert, und einem mit diesem verbundenen Ventilelement, das unter dem Richtungswechsel des Blutdrucks zwischen einer die Durchtrittsöffnung verschließenden Sperrstellung und einer sie freigebenden Öffnungsstellung bewegbar ist und das Blut in der Sperrstellung zu einer zweiten Durchtrittsöffnung gelangen läßt.

Im Stand der Technik sind bereits Herzklappenpro thesen, insbesondere für die Aortenklappe, in vielfältigen Konstruktionsformen und Ausgestaltungen bekannt. Ältere Vorschläge waren auf die Verwendung von Ball-bzw. Deckelklappen-Ventilen als Herzklappenprothese gerichtet, führten jedoch wegen der zwangsläufig auftretenden Umströmungsvorgänge zu einer starken mechanischen Beanspruchung der Blutkörperchen, die deren Beschädigung bzw. Zerstörung fördert und damit die Gefahr von Koagulationen (Thromben) mit sich bringt.

Der Versuch, eine Herzklappenprothese unter Nachahmung der natürlichen Form von Segelklappen zu schaffen ("Medical and Biological Engineering", Juli 1975, Seiten 509 bis 517) - scheitert an Mängeln in der Funktion und/oder Lebensdauer der Prothese.

Deckelklappenprothesen sind beispielsweise aus der DE-PS 15 41 286 und der DE-PS 28 46 299 bekannt, führen aber zu der genannten starken mechanischen Beanspruchung der Blutkörperchen. Aus der DE-OS 27 00 531 ist eine, der natürlichen Aortenklappe nachgebildete Taschenklappe bekannt; diese hat jedoch einen so komplizierten Aufbau, daß sie den Anforderungen an Funktion und Lebensdauer einer Herzklappenprothese, insbesondere einer Aortenklappenprothese nicht gerecht wird.

Der Erfinder der vorliegenden Anmeldung hat zur Überwindung der im Stand der Technik nicht gelösten Probleme eine Herzklappenprothese der eingangs genannten Art vorgeschlagen; diese ist in der DE-PS 34 26 300 offenbart. Aufbau und Funktion dieser bekannten Herzklappenprothese werden in der folgenden Figurenbeschreibung näher erläutert werden.

Obwohl diese Herzklappenprothese die mechanischen Störungen überwindet, an denen der vorgenannte Stand der Technik leidet, ist sie in einzelnen Aspekten noch zu verbessern. Die gattungsbildende Herzklappenprothese besteht aus zwei schlauchförmigen Kanalstücken, die jeweils einen Strömungskanal bilden und deren Wandungen (bzw. eine gemeinsame Wandung) so angeordnet und vom Blutdruck verformbar sind, daß sich der erste Kanal unter dem systolischen, der zweite Kanal unter dem diastolischen Druck im wesentlichen öffnet, während der jeweils andere Kanal geschlossen ist. Der Platzbedarf dieser bekannten Prothese, insbesondere hinsichtlich ihrer Längserstreckung, ist jedoch noch erheblich, was die Implantation erschwert. Außerdem muß zur Erzielung der gewünschten Ventilfunktion das distale schlauchförmige Kanalstück eine Schwenkbewegung durchführen, die unter Biegung der die Strömungskanäle verbindenden Wandung(en) erfolgt. Dies kann nur unter Überwindung von Trägheits-und elastischen Rückstellkräften geschehen, die die Ansprechzeiten der Prothese bei Richtungsumkehr des Blutdruckes relativ lang werden lassen.

Obwohl die Eigenbewegung dieser bekannten Herzklappenprothese gegenüber dem älteren Stand der Technik erheblich verringert ist und den Blutkörperchen nur wenig Prallfläche entgegengesetzt wird, was deren Zerstörung und Koagulation erheblich reduziert, ist die verbleibende Eigenbewegung der Herzklappenprothese daher noch unbefriedigend.

Die Aufgabe der Erfindung besteht vor diesem Hintergrund darin, eine Herzklappenprothese der eingangs genannten Art so zu verbessern, daß ohne Preisgabe der günstigen Durchströmungseigenschaften und langen Lebensdauer eine möglichst widerstandslose Ventilfunktion bei verringerter Gesamtlänge der Prothese ermöglicht wird.

Erfindungsgemäß wird diese Aufgabe dadurch gelöst, daß das Ventilelement von einer flexiblen Zunge gebildet wird, die nur an ihrem einen Endbereich mit einem Umfangs-Teilbereich des Ventilkörpers verbunden ist.

Die Erfindung löst sich von dem Grundkonzept des gattungsbildenden Standes der Technik, nämlich jeden Strömungskanal als - schlauchförmiges Element auszubilden und das Öffnen und Schließen der Kanäle durch Verformung ihrer Wandung zu bewirken. Statt dessen wird gemäß der Erfindung ein einziger, rohrartiger Ventilkörper vorgesehen, der sehr kurz sein kann; die von dem Ventilkörper definierte erste Durchtrittsöffnung wird nicht mehr durch Wandungsverformung des Ventilkörpers, sondern durch eine flexible Zunge gesperrt bzw. freigegeben. Diese Zunge kann aus einem dünnen, kleinen Materialblättchen bestehen, so daß sie sehr viel leichter und schneller bewegt werden kann als der geschlossene, sehr viel größere Schlauch, der beim gattungsbil denden Stand der Technik den diastolisch durchflossenen Strömungskanal bildet.

Natürlich muß auch bei der Erfindung der Blutstrom, den beim gattungsbildenden Stand der Technik der zweite Strömungskanal ableitet, gezielt zu seinem Bestimmungsort geführt werden. Diese Funktion übernimmt gemäß der Erfindung die Zunge, indem sie in ihrer Sperrstellung den Blutstrom zu der zweiten Durchtrittsöffnung gelangen läßt.

Die erfindungsgemäße Herzklappenprothese hat wenigstens die gleiche Lebensdauer wie die gattungsbildende Prothese. Sie setzt den Blutkörperchen nicht mehr Prallfläche entgegen, so daß die durch den gattungsbildenden Stand der Technik bereits erreichten Vorteile auch bei der Erfindung realisiert werden.

Bevorzugte Ausführungsformen der Erfindung sind Gegenstand der Unteransprüche.

Nachstehend werden bevorzugte Ausführungsformen der Erfindung anhand der Zeichnung im einzelnen erläutert. Darin zeigt:

Fig. 1 perspektivisch eine bekannte Prothese;

Fig 2 einen Längsschnitt der Prothese gemäß Fig. 1 entlang der Linien II-II;

Fig. 3 einen Längsschnitt durch die Aorta ascendens, den Arcus aortae und die imlantierte Prothese während der Systole;

Fig. 4 einen Längsschnitt gemäß Fig. 3, jedoch während der Diastole;

Fig. 5 einen Längsschnitt einer ersten Ausführungsform der Erfindung, implantiert in der Aorta ascendens, während der Systole;

Fig. 6 einen Längsschnitt gemäß Fig. 5, während der Diastole;

Fig. 7 einen Längsschnitt einer abgewandelten Prothese ähnlich Fig. 5, während der Systole;

Fig. 8 einen Längsschnitt ähnlich Fig. 7, jedoch während der Diastole;

Fig. 9 einen Teil der Prothese gemäß Fig. 5 und 6, einschließlich der Zunge;

Fig. 10 den Teil gemäß Fig. 9, geschnitten entlang der Linie X-X;

Fig. 11 eine zweite Ausführungsform der Prothese in teilweise geschnittener perspektivischer Ansicht;

Fig. 12 die Prothese gemäß Fig. 11, implantiert in die Aorta ascendens und

Fig. 13 eine schematische Ansicht der Prothese gemäß Fig. 11 und 12 in Blickrichtung durch den Ventilkörper auf die proximale Seite der Zunge.

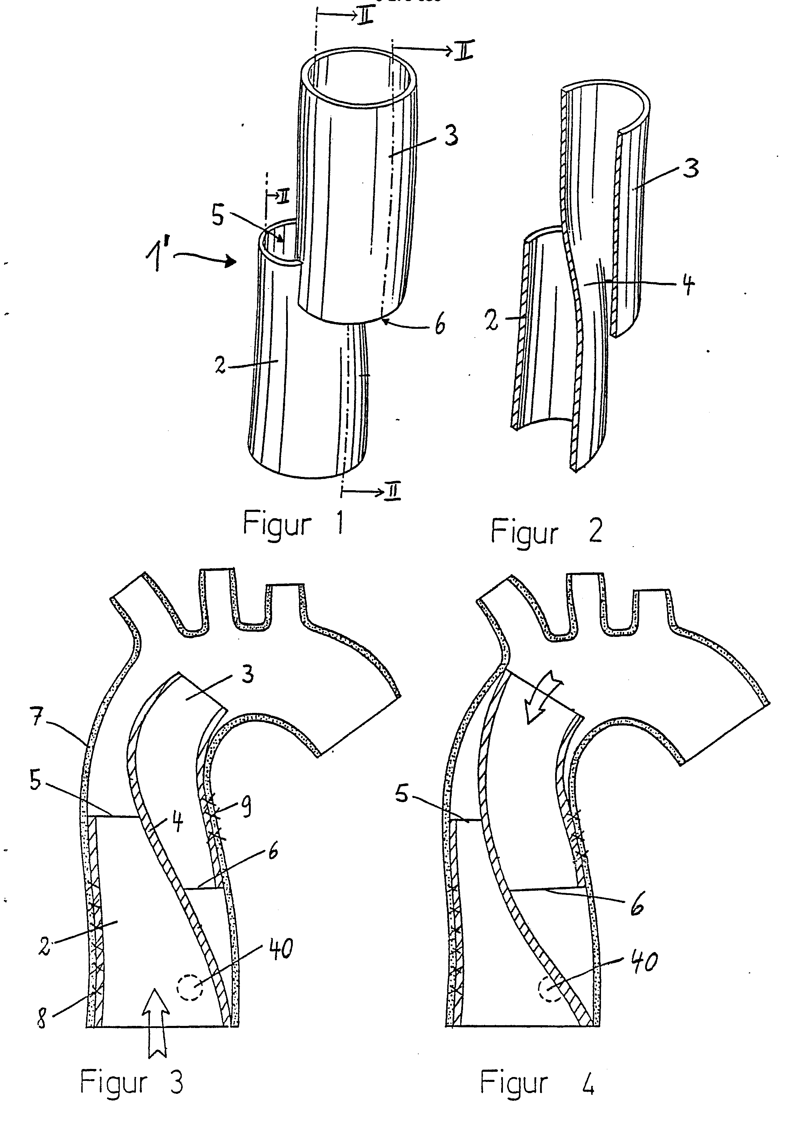

Die in Fig. 1 bis 4 gezeigte, bekannte Herzklappenprothese 1' des Erfinders besteht aus zwei Schlauchelementen 2, 3, die in axialer Richtung gegeneinander verschoben durch eine gemeinsame Wandung 4 verbunden sind. Das erste Schlauchelement 2 bildet eine erste Durchtrittsöffnung 5, das zweite Schlauchelement 3 eine zweite Durchtrittsöffnung 6.

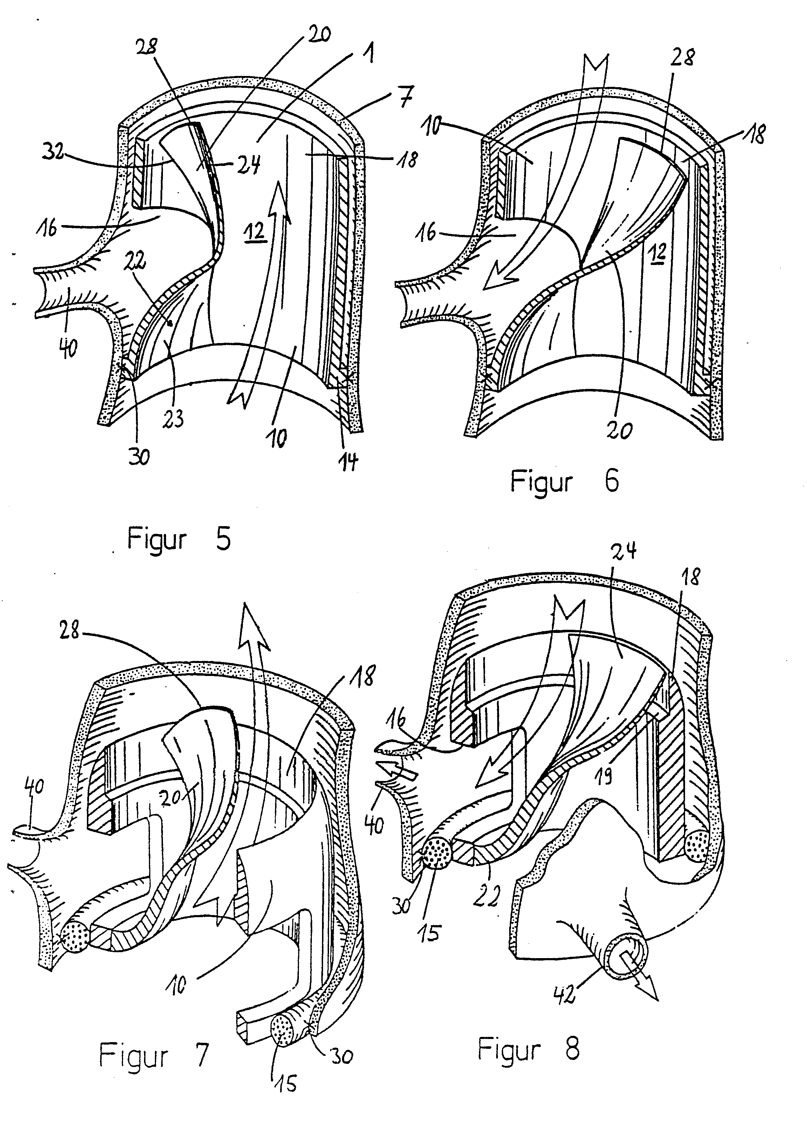

Diese Prothese wird, wie Fig. 3 und 4 zeigen, beispielsweise als Aortenklappenprothese in die Aorta ascendens implantiert, wobei das erste Schlauchelement 2 sich über die Ostien der Koronararterien erstreckt, von denen eines in Fig. 3 und 4 bei 40 angedeutet ist. In dieser Stellung ragt das (vorzugsweise in Längsrichtung gebogene) zweite Schlauchelement 3 bis in den Arcus aortae vor.

Die implantierte Prothese wird durch Stütznähte 8, 9 mit der Aortenwand 7 verbunden.

In der Austreibungsphase gelangt das Schlagvolumen des linken Ventrikels in Pfeilrichtung in das erste Schlauchelement 2 und verläßt dieses durch die erste Durchtrittsöffnung 5. Unter dem systolischen Druck im Schlauchelement 2 wird die gemeinsame Wand 4 der Schlauchelemente 2, 3 aus der Strömungsbahn geschwenkt, so daß der vom zweiten Schlauchelement 3 gebildete Strömungskanal und insbesondere dessen Durchtrittsöffnung 6 geschlossen ist. Umgekehrt strömt das Blut in der diastolischen Phase (Fig. 4) in die entgegengesetzte Richtung, diesmal jedoch durch den am Ende der Austreibungsperiode in den geöffneten Zustand zurückgekehrten Strömungskanal, den das zweite Schlauchelement 3 bildet, wie der Pfeil in Fig. 4 andeutet. Dadurch erweitert sich das zweite Schlauchelement 3 und - schließt die Durchtrittsöffnung 5 des ersten Schlauchelements 2. Das diastolische Blut strömt daher nicht zum Ventrikel, sondern vielmehr durch die zweite Durchtrittsöffnung 6 zum Ostium 40.

Im folgenden wird nun die Erfindung an Beispielen von Aortenklappenprothesen erläutert.

Fig. 5 und 6 zeigen eine erste Ausführungsform der Erfindung. Die gezeigte Herzklappenprothese 1 umfaßt einen Ventilkörper 10, der als kurzes, rohrstückartiges Element ausgebildet ist. Die äußere Form des Ventilkörpers 10 ist der Aortenwandkontur angepaßt.

Am proximalen (in Fig. 5 und 6: unteren) Ende des Ventilkörpers 10 ist dieser zu einem Ringbereich 14 verdickt, der zur Befestigung der Prothese 1 an der Aortenwand 7 durch eine Stütznaht 30 dient.

In der Rohrwandung des Ventilkörpers 10 ist eine Durchbrechung 16 vorgesehen, die bei Implantation der Herzklappenprothese 1 als Aortenklappenprothese dem Ostium 40 einer Koronararterie gegenüberliegt. Dem (nicht gezeigten) Ostium der anderen Koronararterie gegenüberliegend kann eine zweite entsprechende Durchbrechung vorgesehen sein; statt dessen kann die Durchbrechung 16 sich auch in Umfangsrichtung des Ventilkörpers 10 über beide Ostien erstrecken.

Der Wandungsbereich des Ventilkörpers 10 zwischen der Durchbrechung 16 und dem Ringbereich 14 geht in einem Verbindungsbereich 23 einstückig in eine Zunge 20 über, die dadurch an ihrem einen Endbereich 22 mit einem Umfangs-Teilbereich des Ventilkörpers 10 verbunden ist. Die Materialdicke der Zunge 20 verringert sich, ausgehend vom in den Verbindungsbereich 23 übergehenden Endbereich 22 der Zunge 20 bis zu einer im wesentlichen geraden Vorderkante 28 am freien Ende 24 der Zunge. Die Vorderkante 28 geht etwa rechtwinklig in Seitenkanten 32 (nur eine gezeigt) der Zunge 20 über, so daß die Zunge 20 insgesamt etwa rechteckförmig ist.

An seinem distalen (in Fig. 5 und 6: oberen) Ende bildet die Innenumfangswand des Ventilkörpers 10 eine Anlagefläche 18, gegen die sich das freie Ende 24 der Zunge 20 anlegen kann.

Wenn zu Beginn der Systole das Blut vom Ventrikel in die Prothese einströmt, wie der Pfeil in Fig. 5 andeutet, wird das freie Ende 24 der Zunge 20 aus der Strömungsrichtung geschwenkt und legt sich vor die Durchbrechung 16. Das systolische Blut kann daher durch eine erste Durchtrittsöffnung 12 strömen, die von der proximalen Hauptfläche der Zunge 20 und der Innenumfangswand des Ventilkörpers 10 begrenzt ist. Ein unerwünschter Eintritt von systolischem Blut durch die Durchbrechung 16 in das Ostium 40 wird dadurch verhindert, daß die Zunge 20 die Durchbrechung 16 verschließt.

Zu Beginn der diastolischen Phase kehrt sich die Blutdruckrichtung um. Durch die Elastizität des Zungenmaterials ist am Ende der systolischen Phase die Zunge 20 von der in Fig. 5 gezeigten Stellung wieder in Richtung auf die erste Durchtrittsöffnung 12 zurückgeschwenkt worden. Wenn sie jetzt vom diastolischen Blutstrom angeströmt wird, legt sich die Zunge 20, wie Fig. 6 veranschaulicht, sperrend über die erste Durchtrittsöffnung 12 und dichtet die Aorta in Ventrikelrichtung dadurch ab, wobei sich das freie Ende 24 an die Anlagefläche 18 legt. Die distale Hauptfläche der Zunge 20 lenkt den diastolischen Blutstrom zur Durchbrechung 16, so daß er in das Ostium der jeweiligen Koronararterie eintreten kann.

Die in Fig. 7 und 8 gezeigte Abwandlung der soeben beschriebenen Ausführungsform hat einen Befestigungsring (Annähring) 15, der das proximale Ende des Ventilkörpers 10 umschließt. Der Befestigungsring 15 dient analog dem oben beschriebenen Ringbereich 14 zur Anbringung einer Stütznaht 30, die die Prothese an der Aortenwand festlegt.

Bei dieser Ausführungsform ist die Zunge 20 mit einem Endbereich 22 an der Innenseite des proximalen Endes des Ventilkörpers 10 befestigt, beispielsweise durch Schweißen, Kleben oder dergleichen. Die Anlagefläche 18 erstreckt sich bei dieser Ausführungsform zwischen dem distalen Ende des Ventilkörpers 10 und einer einwärts vorspringenden Ringschulter 19, die als Sitz für das freie Ende 24 der Zunge 20 dient.

Wie die Fig. 7 und 8 zeigen, ist die Öffnungs- und Schließfunktion der Zunge bei dieser Ausführungsform analog der oben anhand Fig. 5 und 6 beschriebenen.

Bei beiden Ausführungsformen ist die Breite der Zunge im Verbindungsbereich 23 so groß, daß die Zunge 20 die mit den Ostien 40, 42 kommunizierende Durchbrechung 16 (bzw. zwei separate solche Durchbrechungen 16) überdecken kann. An der Seitenkante 32 kann die Zunge 20 neben der Durchbrechung 16 an der Innenwand des Ventilkörpers 10 befestigt sein, beispielsweise durch Schweißen, Kleben oder einstückiges Anformen. Dies verbessert die Abdeckfunktion der Zunge 20 gegenüber der Durchbrechung 16. Neben der bereits genannten Abnahme der Materialdicke vom Verbindungsbereich 23 zur Vorderkante 28 hin kann die Zunge 20 auch so ausgebildet werden, daß ihre Materialdicke von der Längsmitte zu den Seitenkanten 32 hin abnimmt.

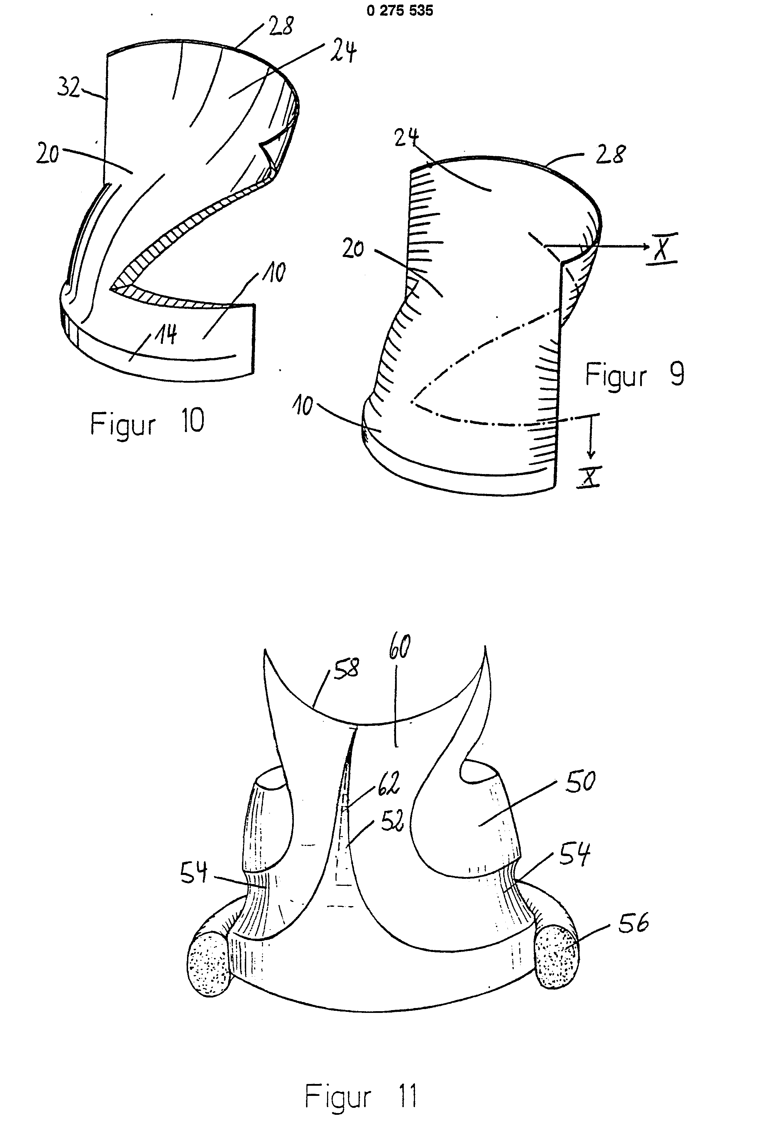

Die Fig. 9 und 10 veranschaulichen die Formgebung der Zunge 20 und zeigen insbesondere die Abnahme der Materialstärke in Richtung auf die Vorderkante 28 und die Seitenkanten 32. In der Praxis wird man diese Bemessungen je nach dem zu verwendenden Material so wählen, daß eine ausreichend große und leichte Beweglichkeit des freien Endes 24 der Zunge 20 einerseits, aber andererseits eine ausreichende elastische Rückstellwirkung erhalten wird, so daß sich die Zunge 20 unter dem Blutdruck schnell und vollständig in die Öffnungs-bzw. Sperrstellung bewegt, aber am Umkehrpunkt zwischen Systole und Diastole nicht in der zuletzt eingenommenen Stellung verharrt, sondern wieder in eine Mittelstellung gebracht wird, von der aus sie dann in die gewünschte neue Stellung treten kann.

Fig. 11 bis 13 zeigen eine zweite Ausführungsform der erfindungsgemäßen Herzklappenprothese. Während bei der oben beschriebenen ersten Ausführungsform die Zunge 20 praktisch vollständig im Inneren des Ventilkörpers 10 liegt, ist bei dieser zweiten Ausführungsform eine Zunge 60 vorgesehen, die am distalen Ende eines Ventilkörpers 50 angeordnet ist.

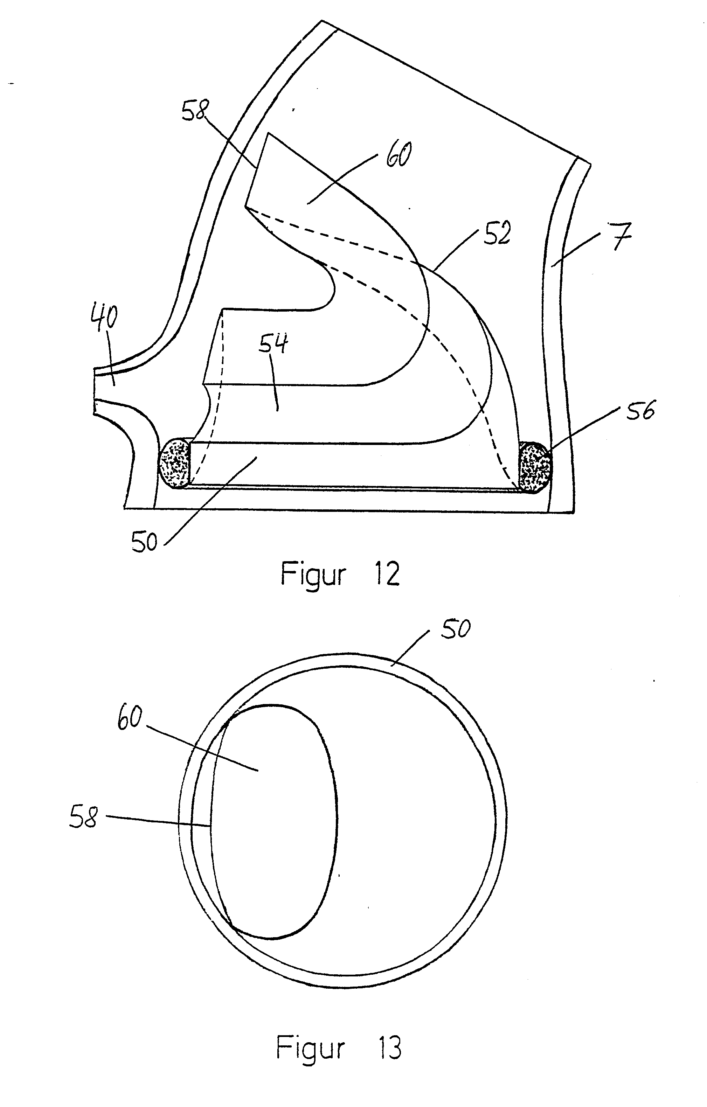

Der Ventilkörper 50 ist auch bei dieser Ausführungsform ein kurzes, rohrstückartiges Element. Er trägt am proximalen (in Fig. 11 und 12: unteren) Ende außenseitig einen Befestigungsring 56, mittels dessen die Prothese an der Aortenwand 7 angenäht werden kann (Fig. 12). Wie auch bei der anhand Fig. 5 bis 10 beschriebenen ersten Ausführungsform, ist dieser Befestigungsring 56 jedoch optional; die Prothese kann auch unmittelbar ohne Ringbereich bzw. Befestigungsring an der Aortenwand 7 festgelegt werden.

Die Zunge 60 geht einstückig aus einem Umfangs-Teilbereich der nicht durchbrochenen Ventilkörperwandung hervor. Sie hat ein freies Ende, das in einer Vorderkante 58 endet, und kann sich mit diesem freien Ende dichtend über die erste Durchtrittsöffnung legen, die von der distalen Öffnung des Ventilkörpers 50 gebildet wird. Wie bei der ersten Ausführungsform wird die Materialdicke der Zunge 60 zur Vorderkante 58 hin geringer. Entlang der Längsmitte 62 der Zunge 60 verläuft eine Erhebung 52, die durch entsprechend größere Materialdicke in diesem Bereich gebildet werden kann. Die Erhebung 52 wird in Richtung auf die Vorderkante 58 hin flacher und schmaler, um die Beweglichkeit des freien Endes der Zunge 60 nicht zu beeinträchtigen.

Die äußere Umfangsfläche des Ventilkörpers 50 ist mit zwei kanalartigen Vertiefungen 54 versehen, die zur distalen, vom Ventilkörper 50 abgewandten Hauptfläche der Zunge 60 führen und jeweils auf einer Seite der Erhebung 52 auf diese Hauptseite der Zunge 60 einmünden. Von der Zunge 60 weg erstrecken sich die Vertiefungen 54 um den Umfang des Ventilkörpers 50 herum, wie Fig. 11 und 12 veranschaulichen.

Wird diese Ausführungsform als Aortenklappenprothese implantiert, wie Fig. 12 zeigt, dann führen die Vertiefungen 54 zu den Ostien der Koronararterien; in Fig. 12 ist dies für ein Ostium 40 dargestellt.

Während der Systole strömt Blut vom Ventrikel zur Prothese und tritt von deren proximaler Seite (in Fig. 12: von unten) in den Ventilkörper 50. Das durchtretende Blut schwenkt das freie Ende der Zunge 60 aus der Strömungsrichtung, wobei die Zunge 60 vom Ventilkörper 50 weggeschwenkt wird.

Am Ende der systolischen Periode schwenkt das freie Ende der Zunge 60 durch die Materialelastizität zurück; bei Richtungsumkehr des Blutdrucks zu Beginn der diastolischen Periode legt sich die Zunge 60 über die Durchtrittsöffnung im Inneren des Ventilkörpers 50. Das anströmende Blut fließt über die distale Hauptfläche der Zunge 60 in die Vertiefungen 54 und gelangt so zu den Ostien. Fig. 13 zeigt diese Stellung der Prothese, gesehen vom Ventrikel aus; die Vertiefungen 54 sind nicht dargestellt.

Die erfindungsgemäßen Prothesen werden so gestaltet, daß sie dem Blutstrom, insbesondere dem systolischen Blutstrom, keine scharfen Kanten entgegensetzen. Dadurch werden nicht nur Turbulenzen, sondern auch mechanische Beschädigungen von Blutkörperchen vermieden.

Die erfindungsgemäßen Prothesen können aus geeigneten Materialien hergestellt werden, die als Implantat verträglich sind und die notwendigen mechanischen Eigenschaften aufweisen. Als biokompatible Kunststoffmaterialien eignen sich hierbei Polyurethan, Polypropylen oder Polytetrafluor ethylen (Teflon). Es kann statt dessen auch daran gedacht werden, geeignete Biomaterialien zu verwenden, beispielsweise Rinderperikard.

Die Anwendung der erfindungsgemäßen Prothese ist nicht auf den Einsatz als Aortenklappenprothese beschränkt; vielmehr läßt sich die Prothese, ggf. unter entsprechender Abwandlung im Aufbau, auch zum Ersatz anderer Organteile als der Aortenklappe verwenden.

Im Aufbau muß die Prothese nicht so ausgebildet werden, daß die Zunge in der Sperrstellung wesentlich am Ventilkörper anliegt; auch eine Anlage an der Gefäßwand (Aortenwand) erfüllt die erfindungsgemäß angestrebte Funktion.

**Ansprüche**

1. Herzklappenprothese in Form eines Zweiwegeventils, mit einem im wesentlichen rohrartigen Ventilkörper, der mindestens eine erste Durchtrittsöffnung definiert, und einem mit diesem verbundenen Ventilelement, das unter dem Richtungswechsel des Blutdrucks zwischen einer die Durchtrittsöffnung verschließenden Sperrstellung und einer sie freigebenden Öffnungsstellung bewegbar ist und das Blut in der Sperrstellung zu einer zweiten Durchtrittsöffnung gelangen läßt, dadurch gekennzeichnet, daß das Ventilelement von einer flexiblen Zunge (20, 60) gebildet wird, die nur an ihrem einen Endbereich (22) mit einem Umfangs-Teilbereich des Ventilkörpers (10, 50) verbunden ist.

2. Herzklappenprothese nach Anspruch 1, dadurch gekennzeichnet, daß die Dicke der Zunge (20, 60) in Richtung auf ihr freies Ende (24) zu abnimmt.

3. Herzklappenprothese nach Anspruch 1 oder 2, dadurch gekennzeichnet, daß die Zunge (20, 60) nahe ihrer Längsmitte (62) dicker ist als an den Seitenkanten (32).

4. Herzklappenprothese nach einem der Ansprüche 1 bis 3, dadurch gekennzeichnet, daß die Zunge (20, 60) zum freien Ende (24) hin breiter wird.

5. Herzklappenprothese nach einem der Ansprüche 1 bis 4, dadurch gekennzeichnet, daß die Zunge (20, 60) an ihrem mit dem Ventilkörper (10, 50) verbundenen Endbereich einstückig mit dem Ventilkörper (10, 50) ausgebildet ist.

6. Herzklappenprothese nach einem .der Ansprüche 1 bis 4,
dadurch gekennzeichnet, daß die Zunge (20) am Ventilkörper (10) angeschweißt, angeklebt o.dgl. ist.

7. Herzklappenprothese nach einem der Ansprüche 1 bis 6,
dadurch gekennzeichnet, daß der Ventilkörper (10) als kurzes, rohrartiges Element ausgebildet ist und sich die Zunge (20) durch den vom Ventilkörper (10) umschlossenen Raum erstreckt.

8. Herzklappenprothese nach Anspruch 7,
dadurch gekennzeichnet, daß der Ventilkörper (10) eine Anlagefläche (18) für das freie Ende (24) der Zunge in deren Sperrstellung aufweist.

9. Herzklappenprothese nach Anspruch 7 oder 8,
dadurch gekennzeichnet, daß der Ventilkörper (10) in seinem mit der Zunge (20) verbundenen Umfangs-Teilbereich wenigstens eine die zweite Durchtrittsöffnung bildende Durchbrechung (16) aufweist.

10. Herzklappenprothese nach Anspruch 9,
dadurch gekennzeichnet, daß die Durchbrechung(en) (16) im als Aortenklappenprothese implantierten Zustand der Herzklappenprothese (1) einerseits mit den Ostien (4c, 42) der Koronararterien und andererseits mit der von der ersten Durchtrittsöffnung (12) abgewandten (distalen) Hauptfläche der Zunge (20) verbunden ist bzw. sind.

11. Herzklappenprothese nach Anspruch 10,
dadurch gekennzeichnet, daß der rohrstückartige Ventilkörper (10) in einem Verbindungsbereich (23), der sich entlang eines die Ostien (40, 42) beider Koronararterien einschließenden Teils der im Einbauzustand proximalen Umfangskante des Ventilkörpers (10) erstreckt, mit dem einen Endbereich (22) der Zunge (20) verbunden ist; daß der Ventilkörper (10) in distaler Richtung auf den Verbindungsbereich folgend wenigstens eine Durchbrechung (16) seiner Rohrwand zur Verbindung mit den Ostien aufweist und an seiner distalen Umfangskante wenigstens in dem Bereich, der nicht oberhalb der Durchbrechung (16) liegt, mit einer Anlagefläche (18) für das freie Ende (24) der Zunge (20) versehen ist, und daß sich die Zunge (20) von dem Verbindungsbereich (23) ausgehend aufwärts in den Innenraum des Ventilkörpers hineinerstreckt und ihre Länge so bemessen ist, daß eine Vorderkante (28) des freien Zungenendes (24) in der Sperrstellung im Bereich der Anlagefläche (18) liegt.

12. Herzklappenprothese nach einem der Ansprüche 1 bis 6,
dadurch gekennzeichnet, daß der Ventilkörper (50) als kurzes, rohrstückartiges Element ausgebildet ist und die Zunge (60) am im implantierten Zustand distalen Endbereich des Ventilkörpers (50) angeordnet ist.

13. Herzklappenprothese nach Anspruch 12,
dadurch gekennzeichnet, daß die Zunge (60) auf ihrer vom Ventilkörper (50) abgewandten (distalen) Hauptfläche eine entlang ihrer Längsmitte (62) verlaufende Erhebung (52) aufweist, die vorzugsweise zum freien Ende der Zunge hin schmaler und flacher wird, und daß der Ventilkörper (50) in seiner Außenwand zwei kanalartige Vertiefungen (54) aufweist, die jeweils an einer Seite der Erhebung auf der distalen Zungenfläche einmünden und die zweite Durchtrittsöffnung bilden und sich jeweils wenigstens über einen Teil der Ventilkörper-Umfangsfläche, zur Verwendung als Aortenklappenprothese bis zu einem Koronararterien-Ostium (40 bzw. 42), erstrecken.

14. Herzklappenprothese nach einem der Ansprüche 1 bis 13,
dadurch gekennzeichnet, daß die Zunge (20, 60) in der Öffnungsstellung die zweite Durchtrittsöffnung (16, 54) im wesentlichen sperrt.

15. Herzklappenprothese nach einem der Ansprüche 1 bis 14,
dadurch gekennzeichnet, daß der Ventilkörper (10, 50), vorzugsweise proximal, einen verdickten Ringbereich (14) oder einen äußeren Befestigungsring (15, 56) (Annähring) aufweist.

16. Herzklappenprothese nach einem der Ansprüche 1 bis 15,
dadurch gekennzeichnet, daß der Ventilkörper (10, 50) in seiner Form der Aortenwand angepaßt ist und vorzugsweise seine vom Blut angeströmten Kanten gerundet sind.

17. Herzklappenprothese nach einem der Ansprüche 1 bis 16,
dadurch gekennzeichnet, daß die Herzklappenprothese (1) aus körperverträglichem Material, insbesondere biokompatiblem Kunststoff, besteht.

0 275 535

Figur 1

Figur 2

Figur 3

Figur 4

Figur 5

Figur 6

Figur 7

Figur 8

**Figur 10**

**Figur 9**

**Figur 11**

0 275 535

Figur 12

Figur 13

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| Y | EP-A-0 183 904 (SHELHIGH INC.) <br> * Figuren 1,9,10; Seite 2, Zeile 34 - Seite 3, Zeile 20; Seite 4, Zeilen 21-37; Seite 5, Zeilen 24-29; Seite 6, Zeilen 1-14,32,33; Seite 9, Zeilen 25-34; Zusammenfassung * <br> --- | 1,2,6,7 ,9,10, 15-17 | A 61 F 2/24 |
| Y,D | DE-A-3 426 300 (BAYKUT) <br> * Figuren 2a,2b,3,5; Seite 9, Zeilen 7-11,21-25 * <br> ----- | 1,2,6,7 ,9,10, 15-17 | |

**RECHERCHIERTE SACHGEBIETE (Int. Cl.4)**

A 61 F

Der vorliegende Recherchenbericht wurde für alle Patentansprüche erstellt

| Recherchenort | Abschlußdatum der Recherche | Prüfer |
|---|---|---|
| DEN HAAG | 30-03-1988 | SEDY, R. |